# EUROPEAN PATENT SPECIFICATION

(11) **EP 3 195 738 B1**
(45) Date of publication and mention of the grant of the patent: **19.09.2018**
(21) Application number: 16155412.6
(22) Date of filing: 12.02.2016
(51) Int. Cl.: A24F 47/00

(54) **ELECTRONIC CIGARETTE HAVING A CERAMIC VAPORIZER**
ELEKTRONISCHE ZIGARETTE MIT EINEM KERAMISCHEN VERDAMPFER
CIGARETTE ÉLECTRONIQUE COMPRENANT UN VAPORISATEUR CÉRAMIQUE

(30) Priority: 20.01.2016 WO PCT/CN2016/071499
(43) Date of publication of application: 26.07.2017
(73) Proprietor: Zhu, Xiaochun, Shenzhen Guangdong 518000 (CN)
(72) Inventor: Zhu, Xiaochun, Shenzhen Guangdong 518000 (CN)
(74) Representative: Weickmann & Weickmann PartmbB

(56) References cited:
- WO-A1-2014/198157
- CN-A- 105 054 308
- CN-U- 204 317 492
- DE-U1-202015 006 397
- US-A1- 2015 272 218
- US-A1- 2016 000 146

## Description

### FIELD

The present invention generally relates to the field of electronic cigarette, and more particularly to electronic cigarettes having ceramic vaporizers.

### BACKGROUND

The background description provided herein is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

It is well known that smoking cigarette is harmful to smoker's health. The active ingredient in a cigarette is mainly nicotine. During smoking, nicotine, along with tar aerosol droplets produced in the cigarette burning, are breathed into the alveolus and absorbed quickly by the smoker. Once nicotine is absorbed into the blood of the smoker, nicotine then produces its effect on the receptors of the smoker's central nervous system, causing the smoker relax and enjoy an inebriety similar to that produced by an exhilarant.

The electronic cigarette is sometimes referred as electronic vaporing device, personal vaporizer (PV), or electronic nicotine delivery system (ENDS). It is a battery-powered device which simulates tobacco smoking. It generally uses a heating element that vaporizes a liquid solution (e-liquid). Some solutions contain a mixture of nicotine and a variety of flavorings, while others release a flavored vapor without nicotine. Many are designed to simulate smoking experience, such as cigarette smoking or cigar smoking. Same of them are made with similar appearance, while others are made considerably different in appearance.

Conventional electronic cigarettes are made with a mouthpiece assembly, a vaporizer assembly, an electric connecting assembly, and an e-liquid storage assembly. The mouthpiece is installed on top of the e-liquid storage assembly, and the vaporizer assembly is installed inside of the e-liquid storage assembly, and electrically connected to a DC power source through the electric connecting assembly. The mouthpiece assembly is connected to the vaporizer assembly and forms an air flow passage. The e-liquid is stored in the e-liquid storage assembly. The e-liquid flows through a vaporizing chamber of the heating assembly using fiber threads. The e-liquid in the fiber threads is then heated by a heating wire of the heating assembly and therefore vaporized. The vaporized e-liquid goes up to the mouthpiece such that a smoker enjoys the vaporized e-liquid. However, when e-liquid makes direct contact with the heating element, it may cause certain burning smell that negatively impact user experiences.
From DE 20 2015 006 397 U1 a ceramic vaporizer for an electronic cigarette is known. The ceramic vaporizer includes a heater which is almost surrounded by a temporary store for an e-liquid, wherein the temporary store has openings enabling the e-liquid to flow to the heater. A conductor of the heater can be a tube having a ceramic layer. Electric cigarettes are also known from US 2016/0000146 A1, CN 204317492, CN 105054308 A, US 2015/0272218 A1, and WO 2014/198157 A1. Therefore, an unaddressed need exists in the art to address the aforementioned deficiencies and inadequacies.

The above-mentioned deficiencies are solved by means of an electronic cigarette according to claim 1.

### SUMMARY

The electronic cigarette according to the present invention, as defined in the claims comprises a cylindrical vaporizer body, and a vaporizer heater. The cylindrical vaporizer body has an interior surface, and an exterior surface. The interior surface forms a central vapor passage and the exterior surface forms an inside wall of an e-liquid storage device. The vaporizer heater is embedded inside the cylindrical vaporizer body.

In certain embodiments, the vaporizer heater may include one or more heating elements. The one or more heating elements are configured to heat e-liquid stored in the e-liquid storage device and the e-liquid is in direct contact with the surfaces of the cylindrical vaporizer body.

In certain embodiments, the one or more heating elements may include: Aluminum (Al), Chromium(Cr), Manganese (Mn), Iron (Fe), Cobalt (Co), Nickel (Ni), Copper (Cu), Zirconium (Zr), Niobium (Nb), Molybdenur (Mo), Rhenium (Re), Silver (Ag), Cadmium (Cd), Tantalum (Ta), Tungsten (W), Iridium (Ir), Platinum (Pt), Gold (Au), and alloys of these elements.

In certain embodiments, the one or more heating elements may include one or more mesh heating elements, and one or more spiral heating elements.

In certain embodiments, each of the one or more heating elements may include a first electrical input terminal configured to connect to a first terminal of a power supply of an electronic cigarette, and a second electrical input terminal configured to connect to a second terminal of the power supply of the electronic cigarette.

In one embodiment, the e-liquid storage device may include an e-liquid storage tank positioned outside of the exterior surface. In another embodiment, the e-liquid storage device may include a cylindrical e-liquid storage medium positioned outside of the exterior surface. The cylindrical e-liquid storage medium may include cotton fibers, polypropylene fibers, terylene fibers, nylon fibers, porous ceramic materials, and any combinations of these materials.

The electronic cigarette according to the present invention, as defined in the claims comprises the ceramic vaporizer described above.

As indicated above, the present invention relates to an electronic cigarette having a ceramic vaporizer which includes a cylindrical vaporizer body, and a vaporizer heater, wherein the cylindrical vaporizer body includes an interior surface, and an exterior surface. The interior surface forms a central vapor passage, and the exterior surface forms an inside wall of an e-liquid storage device. The vaporizer heater is embedded inside the cylindrical vaporizer body.

In certain embodiments, the vaporizer heater may include one or more heating elements. The one or more heating elements are configured to heat e-liquid stored in the e-liquid storage device and the e-liquid is in direct contact with the surfaces of the cylindrical vaporizer body.

In certain embodiments, the one or more heating elements may include Aluminum (Al), Chromium(Cr), Manganese (Mn), Iron (Fe), Cobalt (Co), Nickel (Ni), Copper (Cu), Zirconium (Zr), Niobium (Nb), Molybdenur (Mo), Rhenium (Re), Silver (Ag), Cadmium (Cd), Tantalum (Ta), Tungsten (W), Iridium (Ir), Platinum (Pt), Gold (Au), and alloys of these elements.

In certain embodiments, the one or more heating elements may include one or more mesh heating elements, and one or more spiral heating elements.

In certain embodiments, each of the one or more heating elements may include a first electrical input terminal configured to connect to a first terminal of a power supply of the electronic cigarette, and a second electrical input terminal configured to connect to a second terminal of the power supply of the electronic cigarette.

The electronic cigarette according to the invention includes an air adjustment assembly having an air adjustment assembly base, an air adjustment ring, and a sealing ring. The air adjustment assembly base includes an upper tubular portion threadedly connected to a lower outside threaded portion of the ceramic vaporizer. An air adjustment chamber is defined inside of the air adjustment assembly base. The air adjustment chamber includes a predetermined number of first air vents provided to supply air from outside to inside of the air adjustment chamber. The air adjustment ring is positioned outside of the upper tubular portion of the air adjustment assembly base. The air adjustment ring includes a predetermined number of second air vents. The sealing ring is configured to prevent air leak from the air adjustment chamber.

Consequently, when a user rotates the air adjustment ring around the upper tubular portion of the air adjustment assembly base, and when the locations of the second air vents of the air adjustment ring match the locations of the first air vents, air flow from outside to the air adjustment chamber reaches maximum capacity. When the user further rotates the air adjustment ring around the upper tubular portion, the air flow decreases. When the locations of the second air vents of the air adjustment ring completely misalign with the locations of the first air vents, the air flow stops.

In certain embodiments, the electronic cigarette may also include an electric connector assembly. The electric connector assembly may include an electric connector base, an electrode, and an insulation cover. The electric connector base is attached to the air adjustment assembly and adapted for connecting the electric power supply to the one or more heating elements of the ceramic vaporizer. The electric connector base may include an outer thread configured to electrically connect the first terminal of the power supply of the electronic cigarette to the first electrical input terminal of the one or more heating elements. The electrode is configured to electrically connect the second terminal of the power supply to the second electrical input terminal of one or more heating elements. The insulation cover is positioned between the electric connector base and the electrode to provide insulation between the first terminal and the second terminal of the power supply of the electronic cigarette.

In one embodiment, the electronic cigarette may also include an electric power switch configured to allow the user to turn on and off the power supply to the electronic cigarette.

In certain embodiments, the electronic cigarette may also include an electric power adjustment device. The electric power adjustment device may allow the user to adjust the electric power to control vaporization of the e-liquid.

In certain embodiments, the e-liquid storage device may include an e-liquid storage tank positioned outside of the exterior surface of the ceramic vaporizer. In another embodiment, the e-liquid storage device may include a cylindrical e-liquid storage medium positioned outside of the exterior surface. The cylindrical e-liquid storage medium may include cotton fibers, polypropylene fibers, terylene fibers, nylon fibers, porous ceramic materials, and any combinations of these materials.

In certain embodiments, the electronic cigarette may also include a mouthpiece assembly. The mouth piece assembly may include a mouthpiece, a mouthpiece connector, and a mouthpiece sealing ring. The mouthpiece may include a hollow center air passage connected to the central vapor passage of the ceramic vaporizer. The hollow center air passage is configured to provide vaporized e-liquid to a user. The mouthpiece connector may include a threaded lower portion threadedly connected to a ceramic vaporizer body support. The mouthpiece sealing ring is configured to prevent the vaporized e-liquid to leak from the mouthpiece connector.

These and other aspects of the present invention will become apparent from the following description of the preferred embodiment taken in conjunction with the following drawings.

### BRIEF DESCRIPTION OF THE DRAWINGS

The accompanying drawings illustrate one or more embodiments of the invention and, together with the written description, serve to explain the principles of the invention. Wherever possible, the same reference numbers are used throughout the drawings to refer to the same or like elements of an embodiment. The drawings do not limit the present invention to the specific embodiments disclosed and described herein. The drawings are not necessarily to scale, emphasis instead being placed upon clearly illustrating the principles of the invention, and wherein:
FIG. 1 is a cross-sectional view of a ceramic vaporizer according to certain embodiments of the present invention;
FIG. 2 is a cross-sectional view of another ceramic vaporizer having a cylindrical e-liquid storage medium according to certain embodiments of the present invention;
FIG. 3 is a perspective view of an electronic cigarette having a ceramic vaporizer according to certain embodiments of the present invention;
FIG. 4 is an exploded view of the electronic cigarette having the ceramic vaporizer as shown in FIG. 3 according to certain embodiments of the present invention; and
FIG. 5 is a cross-sectional view of the electronic cigarette having the ceramic vaporizer as shown in FIG. 3 according to certain embodiments of the present invention.

### DETAILED DESCRIPTION

The present invention will now be described more fully hereinafter with reference to the accompanying drawings, in which exemplary embodiments of the invention are shown.

As used herein, "around", "about" or "approximately" shall generally mean within 20 percent, preferably within 10 percent, and more preferably within 5 percent of a given value or range. Numerical quantities given herein are approximates, meaning that the term "around", "about" or "approximately" can be inferred if not expressly stated.

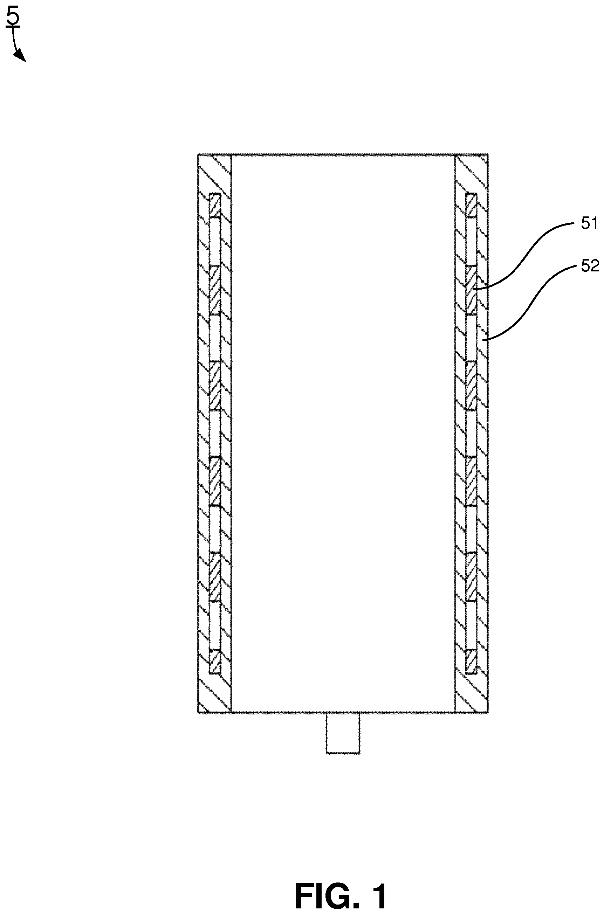

The description will be made as to the embodiments of the present invention in conjunction with the accompanying drawings FIGs. 1 through 5. In accordance with the purposes of this invention, as embodied and broadly described herein, this invention relates to an electronic cigarette 10, comprising a ceramic vaporizer 5.

The electronic cigarette according to the present invention comprises a ceramic vaporizer 5 for electronic cigarette. Referring now to FIG. 1, a cross-sectional view of a ceramic vaporizer 5 is shown according to certain embodiments of the present invention. The ceramic vaporizer 5 may include a cylindrical vaporizer body 52, and a vaporizer heater 51. The cylindrical vaporizer body 52 has an interior surface, and an exterior surface. The interior surface forms a central vapor passage and the exterior surface forms an inside wall of an e-liquid storage device. The ceramic vaporizer 5 is made of high quality ceramic material. It have very strong and durable surface. The vaporizer heater 51 is embedded inside the cylindrical vaporizer body 52. In certain embodiments, the vaporizer heater 51 may include one or more heating elements. The one or more heating elements are configured to heat e-liquid stored in the e-liquid storage device and the e-liquid is in direct contact with the surfaces of the cylindrical vaporizer body 52. The e-liquid is heated through the exterior surface of the ceramic vaporizer 5. This configuration avoid direct contact between e-liquid and heating elements and it may prevent burning smell when the e-liquid makes direct contact with the heating elements. The one or more heating elements are totally covered by the ceramic material and therefore it is safe and may prevent short circuit. The ceramic vaporizer 5 is formed in much higher temperature than it is actually used in the electronic cigarette, therefore it is safe to use. The heat is transferred evenly on the surface of the ceramic vaporizer 5. On one hand, the heating elements heat the ceramic vaporizer quickly. On the other hand, the ceramic vaporizer 5 may be cooled quickly as well. The heating elements are not exposed to air so that the heating elements are prevented from oxidation. This configuration also prolongs the life of the ceramic vaporizer 5 and its heating elements.

In certain embodiments, the one or more heating elements may include: Aluminum (Al), Chromium(Cr), Manganese (Mn), Iron (Fe), Cobalt (Co), Nickel (Ni), Copper (Cu), Zirconium (Zr), Niobium (Nb), Molybdenur (Mo), Rhenium (Re), Silver (Ag), Cadmium (Cd), Tantalum (Ta), Tungsten (W), Iridium (Ir), Platinum (Pt), Gold (Au), and alloys of these elements.

In certain embodiments, the one or more heating elements may include certain electrothermal alloys such as Nickel Chromium alloys, OCr25AL5, OCr27AL7MO2, OCr21AL6Nb, Crl5Ni60, Cr20Ni80, and Ocrl9AL3 etc.

In one embodiment, the one or more heating elements may include heating wires wounded in mesh form. In another embodiment, the one or more heating elements may include heating wires wounded in spiral form.

In certain embodiments, each of the one or more heating elements may include a first electrical input terminal configured to connect to a first terminal of a power supply of an electronic cigarette, and a second electrical input terminal configured to connect to a second terminal of the power supply of the electronic cigarette.

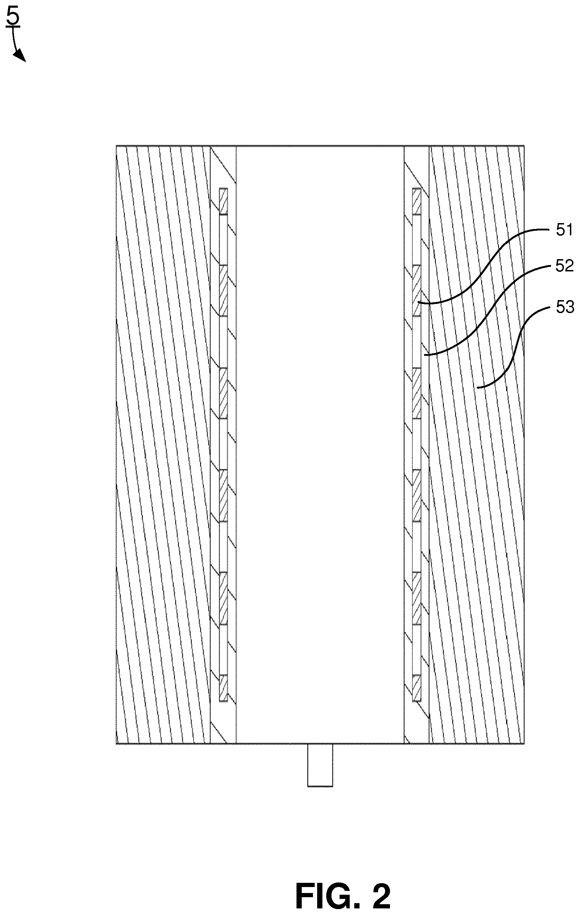

In one embodiment, the e-liquid storage device may include an e-liquid storage tank positioned outside of the exterior surface. In another embodiment, the e-liquid storage device may include a cylindrical e-liquid storage medium 53 positioned outside of the exterior surface, as shown in FIG. 2. The cylindrical e-liquid storage medium 53 may include cotton fibers, polypropylene fibers, terylene fibers, nylon fibers, porous ceramic materials, and any combinations of these materials.

In certain embodiments, the present invention relates to an electronic cigarette having the ceramic vaporizer described above.

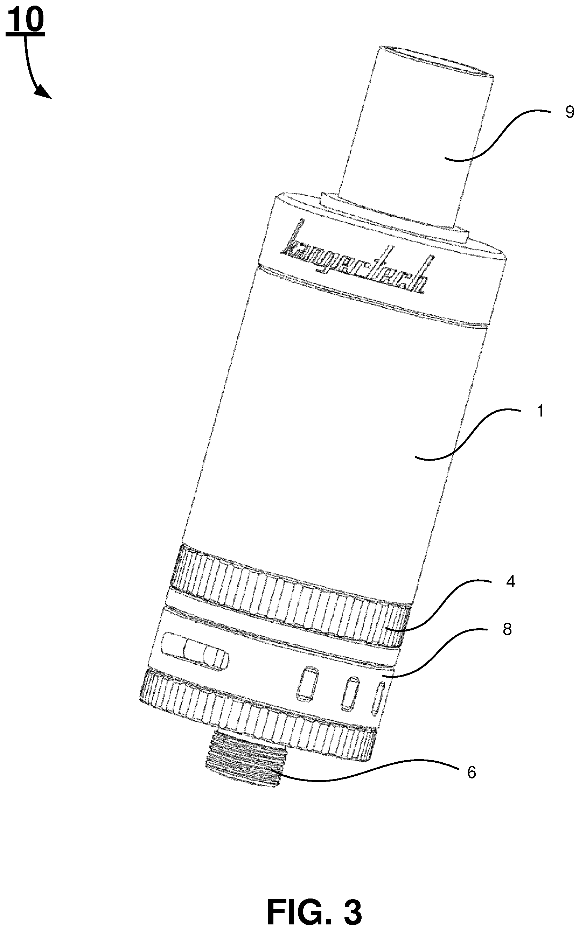

The present invention relates to an electronic cigarette 10 having a ceramic vaporizer 5. Referring now to FIGs. 3, 4, and 5, a perspective view, an exploded view and a cross-sectional view of the electronic cigarette 10 having the ceramic vaporizer 5 are shown, respectively, according to certain embodiments of the present invention. The electronic cigarette 10 may include a mouthpiece assembly 9, an electronic cigarette body 1, a lower portion 4 of the ceramic vaporizer 5, an air adjustment assembly 8, and an electric connector assembly 6. In certain embodiments, the mouthpiece assembly 9 may be removed to refill e-liquid to e-liquid storage of the electronic cigarette 10. The electronic cigarette body 1 may include a top cover 12, and a tubular electronic cigarette body 11. In certain embodiments, the tubular electronic cigarette body 11 may define one or more openings to allow a user to observe level of e-liquid inside of an e-liquid storage device.

The electronic cigarette 10 includes the ceramic vaporizer 5. The ceramic vaporizer 5 includes a cylindrical vaporizer body 52, and a vaporizer heater 51. The cylindrical vaporizer body 52 includes an interior surface, and an exterior surface. The interior surface forms a central vapor passage, and the exterior surface forms an inside wall of an e-liquid storage device. The vaporizer heater 51 is embedded inside the cylindrical vaporizer body.

In certain embodiments, the vaporizer heater 51 may include one or more heating elements. The one or more heating elements are configured to heat e-liquid stored in the e-liquid storage device and the e-liquid is in direct contact with the surfaces of the cylindrical vaporizer body 52.

In certain embodiments, the one or more heating elements may include Aluminum (Al), Chromium(Cr), Manganese (Mn), Iron (Fe), Cobalt (Co), Nickel (Ni), Copper (Cu), Zirconium (Zr), Niobium (Nb), Molybdenur (Mo), Rhenium (Re), Silver (Ag), Cadmium (Cd), Tantalum (Ta), Tungsten (W), Iridium (Ir), Platinum (Pt), Gold (Au), and alloys of these elements.

In certain embodiments, the one or more heating elements may include one or more mesh heating elements, and one or more spiral heating elements.

In certain embodiments, each of the one or more heating elements may include a first electrical input terminal configured to connect to a first terminal of a power supply of the electronic cigarette, and a second electrical input terminal configured to connect to a second terminal of the power supply of electronic cigarette.

In certain embodiments, the electronic cigarette 10 may also include a mouthpiece assembly 9. The mouth piece assembly 9 may include a mouthpiece, a mouthpiece connector, and a mouthpiece sealing ring. The mouthpiece may include a hollow center air passage connected to the central vapor passage of the ceramic vaporizer 5. The hollow center air passage is configured to provide vaporized e-liquid to a user. The mouthpiece connector may include a threaded lower portion threadedly connected to a ceramic vaporizer body support. The mouthpiece sealing ring is configured to prevent the vaporized e-liquid to leak from the mouthpiece connector.

The electronic cigarette 10 includes an air adjustment assembly 8. The air adjustment assembly 8 includes an air adjustment assembly base, an air adjustment ring, and a sealing ring. The air adjustment assembly base includes an upper tubular portion threadedly connected to a lower outside threaded portion of the ceramic vaporizer 5. An air adjustment chamber is defined inside of the air adjustment assembly base. The air adjustment chamber includes a predetermined number of first air vents provided to supply air from outside to inside of the air adjustment chamber. The air adjustment ring is positioned outside of the upper tubular portion of the air adjustment assembly base. The air adjustment ring includes a predetermined number of second air vents. The sealing ring is configured to prevent air leak from the air adjustment chamber.

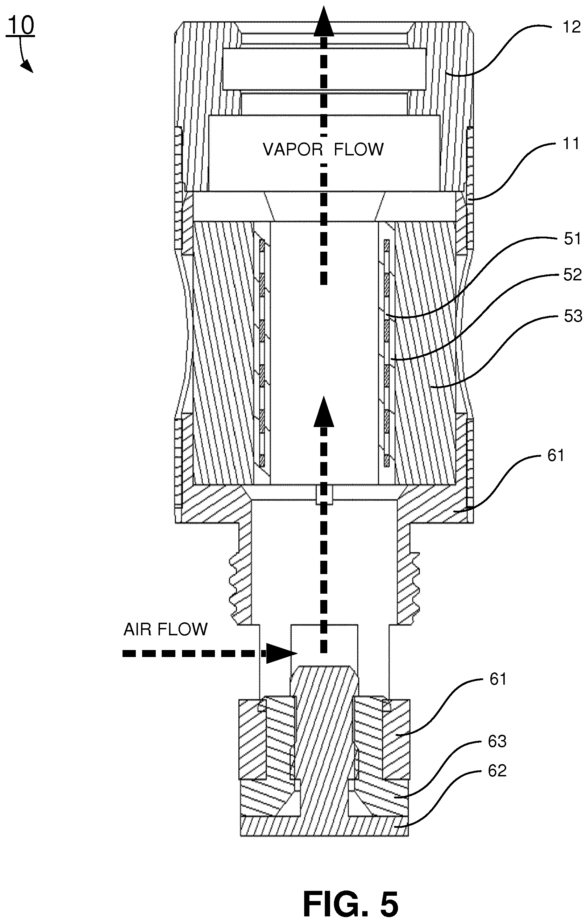

When a user rotates the air adjustment ring around the upper tubular portion of the air adjustment assembly base, and when the locations of the second air vents of the air adjustment ring match the locations of the first air vents, air flow from outside to the air adjustment chamber reaches maximum capacity as indicated by a dashed arrow in FIG. 5. The air inside the air adjustment chamber may flow upwards when the user sucks air through the mouthpiece assembly 9 as indicated by the upward arrow in FIG. 5. When the air pass through the central vapor passage, the air becomes vapor and the vapor flows upward to the user through the mouthpiece assembly 9. When the user further rotates the air adjustment ring around the upper tubular portion, the air flow decreases. When the locations of the second air vents of the air adjustment ring completely misalign with the locations of the first air vents, the air flow stops.

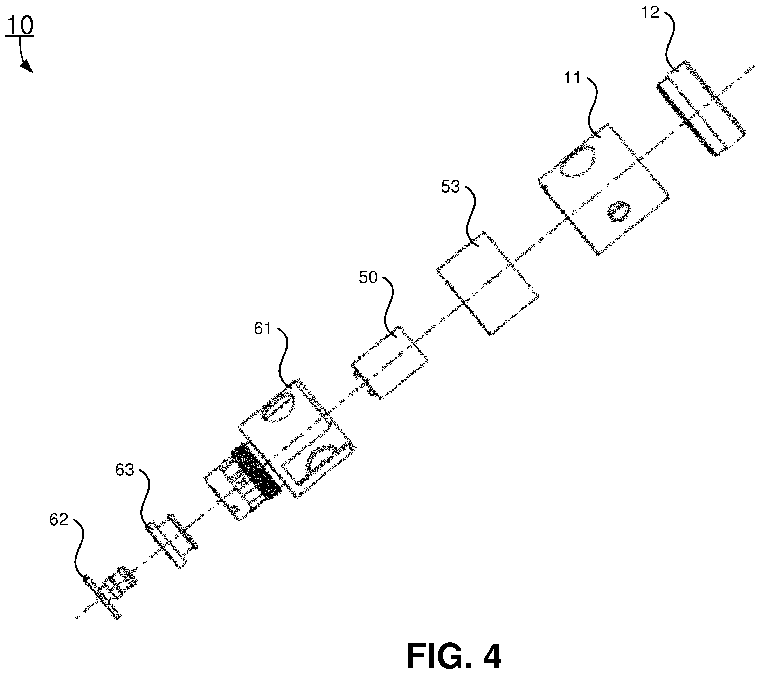

In certain embodiments, the electronic cigarette 10 may also include an electric connector assembly 6. The electric connector assembly 6 may include an electric connector base 61, an electrode 62, and an insulation cover 63, as shown in FIGs. 4 and 5. The electric connector base is attached to the air adjustment assembly 8 and adapted for connecting the electric power supply to the one or more heating elements of the ceramic vaporizer 5. The electric connector base may include an outer thread configured to electrically connect the first terminal of the power supply of the electronic cigarette to the first electrical input terminal of the one or more heating elements. The electrode is configured to electrically connect the second terminal of the power supply to the second electrical input terminal of one or more heating elements. The insulation cover is positioned between the electric connector base and the electrode to provide insulation between the first terminal and the second terminal of the power supply of the electronic cigarette.

In one embodiment, the electronic cigarette 10 may also include an electric power switch configured to allow the user to turn on and off the power supply to the electronic cigarette.

In certain embodiments, the electronic cigarette 10 may also include an electric power adjustment device. The electric power adjustment device may allow the user to adjust the electric power to control vaporization of the e-liquid.

In certain embodiments, the e-liquid storage device may include an e-liquid storage tank positioned outside of the exterior surface of the ceramic vaporizer. In another embodiment, the e-liquid storage device may include a cylindrical e-liquid storage medium 53 positioned outside of the exterior surface as shown in FIGs. 4 and 5. The cylindrical e-liquid storage medium 53 may include cotton fibers, polypropylene fibers, terylene fibers, nylon fibers, porous ceramic materials, and any combinations of these materials.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to activate others skilled in the art to utilize the invention and various embodiments and with various modifications as are suited to the particular use contemplated. Alternative embodiments will become apparent to those skilled in the art to which the present invention pertains without departing from its spirit and scope. Accordingly, the scope of the present invention is defined by the appended claims, the foregoing description and the exemplary embodiments described therein, and accompanying drawings.

The present invention relates to an electronic cigarette having a ceramic vaporizer including a cylindrical vaporizer body, and a vaporizer heater. The cylindrical vaporizer body has an interior surface, and an exterior surface. The interior surface forms a central vapor passage and the exterior surface forms an inside wall of an e-liquid storage device. The vaporizer heater is embedded inside the cylindrical vaporizer body. The vaporizer heater may include one or more heating elements configured to heat e-liquid stored in e-liquid storage device and e-liquid is in direct contact with surfaces of the cylindrical vaporizer body. The one or more heating elements may include certain electrothermal alloys. In certain embodiments, the one or more heating elements may include one or more mesh heating elements, and one or more spiral heating elements.

## Claims

1. An electronic cigarette (10) comprising
a ceramic vaporizer (5) comprising:
- a cylindrical vaporizer body (52) having an interior surface, and an exterior surface, wherein the interior surface forms a central vapor passage, and the exterior surface is suitable for forming an inside wall of an e-liquid storage device; and
- a vaporizer heater (51) embedded inside the cylindrical vaporizer body,
**characterized in that** the electronic cigarette (10) further comprises an air adjustment assembly (8) having:
- an air adjustment assembly base having an upper tubular portion threadedly connected to a lower outside threaded portion of the ceramic vaporizer, an air adjustment chamber defined inside of the air adjustment assembly base, and a plurality of first air vents;
- an air adjustment ring disposed outside of the upper tubular portion of the air adjustment assembly base, wherein the air adjustment ring comprises a plurality of second air vents; and
- a sealing ring configured to prevent air leak from the air adjustment assembly (8),
- wherein when a user rotates the air adjustment ring around the upper tubular portion of the air adjustment assembly base, and when the locations of the plurality of second air vents of the air adjustment ring match the locations of the plurality of first air vents, air flow from outside to the air adjustment chamber reaches maximum capacity, and when the user further rotates the air adjustment ring around the upper tubular portion, the air flow decreases, and when the locations of the plurality of second air vents of the air adjustment ring completely misalign with the locations of the plurality of first air vents, the air flow stops.

2. The electronic cigarette (10) of claim 1, further comprising an electric connector assembly (6), wherein the electric connector assembly (6) comprises:
an electric connector base (61) attached to the air adjustment assembly (8) and adapted for connecting the power supply to the one or more heating elements of the ceramic vaporizer, wherein the electric connector base (61) comprises an outer thread configured to electrically connect a first terminal of the power supply of the electronic cigarette to the first electrical input terminal of the one or more heating elements;
an electrode (62) configured to electrically connect the second terminal of the power supply to the second electrical input terminal of one or more heating elements; and
an insulation cover (63) positioned between the electric connector base (61) and the electrode (62) to provide insulation between the first terminal and the second terminal of the power supply of the electronic cigarette.

3. The electronic cigarette (10) of claim 1 or 2, further comprising an electric power switch configured to allow the user to turn on and off the power supply to the electronic cigarette (10).

4. The electronic cigarette (10) of one of claims 1 to 3, further comprising an electric power adjustment device configured to allow the user to adjust the electric power to control vaporization of the e-liquid.

5. The electronic cigarette (10) of one of claims 1 to 4, further comprising a mouthpiece assembly (9) having:
a mouthpiece having a hollow center air passage connected to the central vapor passage of the ceramic vaporizer (5) and configured to provide vaporized e-liquid to a user;
a mouthpiece connector having a threaded lower portion threadedly connected to a ceramic vaporizer body support; and
a mouthpiece sealing ring configured to prevent the vaporized e-liquid to leak from the mouthpiece connector.

6. The electronic cigarette (10) of one of claims 1 to 5, wherein the vaporizer heater (51) comprises one or more heating elements configured to heat e-liquid in direct contact with the surfaces of the cylindrical vaporizer body (52).

7. The electronic cigarette (10) of claim 6, wherein the one or more heating elements comprise:
one or more mesh heating elements; and
one or more spiral heating elements.

8. The electronic cigarette (10) of one of claim 6 or 7, wherein each of the one or more heating elements comprises:
a first electrical input terminal configured to be connect to a first terminal of a power supply of an electronic cigarette; and
a second electrical input terminal configured to connect to a second terminal of the power supply of the electronic cigarette.

9. The electronic cigarette (10) of one of claims 1 to 8, wherein the e-liquid storage device comprises an e-liquid storage tank outside of the exterior surface.

10. The electronic cigarette (10) of claim 1, wherein the e-liquid storage device comprises a cylindrical e-liquid storage medium (53) positioned outside of the exterior surface.

## Patentansprüche

1. Elektronische Zigarette (10), umfassend:
einen keramischen Verdampfer (5), umfassend:
- einen zylindrischen Verdampferkörper (52), welcher eine Innenfläche und einer Außenfläche aufweist, wobei die Innenfläche einen zentralen Dampfdurchgang bildet und die Außenfläche zum Bilden einer Innenwand einer e-Flüssigkeit-Speichervorrichtung geeignet ist; und
- eine Verdampfer-Heizvorrichtung (51), welche im Inneren des zylindrischen Verdampferkörpers eingebettet ist,
**dadurch gekennzeichnet, dass** die elektronische Zigarette (10) ferner eine Lufteinstellanordnung (8) umfasst, welche aufweist:
- eine Lufteinstellanordnungsbasis, welche einen oberen röhrenförmigen Abschnitt, welcher mittels eines Gewindes mit einem unteren Außenseitengewindeabschnitt des keramischen Verdampfers verbunden ist, eine im Inneren der Lufteinstellanordnungsbasis definierte Lufteinstellkammer und eine Mehrzahl erster Lufteinlässe aufweist;
- einen Lufteinstellring, welcher außerhalb des oberen röhrenförmigen Abschnitts der Lufteinstellanordnungsbasis angeordnet ist, wobei der Lufteinstellring eine Mehrzahl zweiter Lufteinlässe umfasst; und
- einen Dichtring, welcher dazu eingerichtet ist, einen Luftaustritt aus der Lufteinstellanordnung (8) zu verhindern,
- wobei ein Luftstrom von außerhalb der Lufteinstellkammer eine maximale Kapazität erreicht, wenn ein Bediener den Lufteinstellring um den oberen röhrenförmigen Abschnitt der Lufteinstellanordnungsbasis dreht und wenn die Positionen der Mehrzahl zweiter Lufteinlässe des Lufteinstellrings mit den Positionen der Mehrzahl erster Lufteinlässe übereinstimmen, und die Luftströmung abnimmt, wenn der Bediener den Lufteinstellring weiter um den oberen röhrenförmigen Abschnitt dreht, und die Luftströmung stoppt, wenn die Positionen der Mehrzahl zweiter Lufteinlässe des Lufteinstellrings vollständig versetzt zu den Positionen der Mehrzahl erster Lufteinlässe sind.

2. Elektronische Zigarette (10) nach Anspruch 1, ferner umfassend eine elektrische Verbindungsanordnung (6), wobei die elektrische Verbindungsanordnung (6) umfasst:
eine elektrische Verbinderbasis (61), welche an der Lufteinstellanordnung (8) angebracht ist und zum Verbinden der Leistungszufuhr mit dem einen oder den mehreren Heizelementen des keramischen Verdampfers eingerichtet ist, wobei die elektrische Verbinderbasis (61) ein äußeres Gewinde umfasst, welches dazu eingerichtet ist, einen ersten Anschluss der Leistungszufuhr der elektronischen Zigarette mit dem ersten elektrischen Eingangsanschluss des einen oder der mehreren Heizelemente elektrisch zu verbinden;
eine Elektrode (62), welche dazu eingerichtet ist, den zweiten Anschluss der Leistungszufuhr mit dem zweiten elektrischen Eingangsanschluss eines oder mehrerer Heizelemente elektrisch zu verbinden; und
eine Isolierabdeckung (63), welche zwischen der elektrischen Verbindungsbasis (61) und der Elektrode (62) positioniert ist, um eine Isolierung zwischen dem ersten Anschluss und dem zweiten Anschluss der Leistungszufuhr der elektronischen Zigarette bereitzustellen.

3. Elektronische Zigarette (10) nach Anspruch 1 oder 2, ferner umfassend einen elektrischen Leistungsschalter, welcher dazu eingerichtet ist, es dem Bediener zu erlauben, die Leistungszufuhr zu der elektronischen Zigarette (10) an und aus zu schalten.

4. Elektronische Zigarette (10) nach einem der Ansprüche 1 bis 3, ferner umfassend eine elektrische Leistungseinstellvorrichtung, welche dazu eingerichtet ist, es dem Bediener zu erlauben, die elektrische Leistung zum Regeln einer Verdampfung der e-Flüssigkeit einzustellen.

5. Elektronische Zigarette (10) nach einem der Ansprüche 1 bis 4, ferner umfassend eine Mundstückanordnung (9), welche aufweist:
ein Mundstück, welches einen hohlen zentralen Luftdurchgang aufweist, welcher mit dem zentralen Dampfdurchgang des keramischen Verdampfers (5) verbunden ist und dazu eingerichtet ist, einem Bediener verdampfte e-Flüssigkeit bereitzustellen;
einen Mundstückverbinder, welcher einen unteren Gewindeabschnitt aufweist, welcher mittels eines Gewindes mit einem keramischen Verdampferkörperhalter verbunden ist; und
einen Mundstückdichtring, welcher dazu eingerichtet ist, einen Austritt der verdampften e-Flüssigkeit aus dem Mundstückverbinder zu verhindern.

6. Elektronische Zigarette (10) nach einem der Ansprüche 1 bis 5, wobei die Verdampferheizvorrichtung (51) ein oder mehrere Heizelemente umfasst, welche dazu eingerichtet sind, e-Flüssigkeit in direktem Kontakt mit den Flächen des zylindrischen Verdampferkörpers (52) zu erwärmen.

7. Elektronische Zigarette (10) nach Anspruch 6, wobei das eine oder die mehreren Heizelemente umfassen:
ein oder mehrere Netz-Heizelemente; und
ein oder mehrere Spiral-Heizelemente.

8. Elektronische Zigarette (10) nach einem der Ansprüche 6 oder 7, wobei jedes aus dem einen oder den mehreren Heizelementen umfasst:
einen ersten elektrischen Eingangsanschluss, welcher dazu eingerichtet ist, mit einem ersten Anschluss einer Leistungszufuhr einer elektronischen Zigarette verbunden zu sein; und
einen zweiten elektrischen Eingangsanschluss, welcher dazu eingerichtet ist, mit einem zweiten Anschluss der Leistungszufuhr der elektronischen Zigarette verbunden zu sein.

9. Elektronische Zigarette (10) nach einem der Ansprüche 1 bis 8, wobei die e-Flüssigkeit-Speichervorrichtung einen e-Flüssigkeit-Speichertank außerhalb der Außenfläche umfasst.

10. Elektronische Zigarette (10) nach Anspruch 1, wobei die e-Flüssigkeit-Speichervorrichtung ein zylindrisches e-Flüssigkeit-Speichermedium (53) umfasst, welches außerhalb der Außenfläche positioniert ist.

## Revendications

1. Cigarette électronique (10) comprenant
un vaporisateur céramique (5) comprenant :
- un corps de vaporisateur cylindrique (52) ayant une surface intérieure, et une surface extérieure, dans laquelle la surface intérieure forme un passage de vapeur central, et la surface extérieure convient pour former une paroi intérieure d'un dispositif de stockage de liquide à vapoter ; et
- un chauffage de vaporisateur (51) encastré à l'intérieur du corps de vaporisateur cylindrique,
**caractérisée en ce que** la cigarette électronique (10) comprend en outre un ensemble réglage d'air (8) comportant :
- une base d'ensemble réglage d'air ayant une portion tubulaire supérieure raccordée par filets à une portion filetée extérieure inférieure du vaporisateur céramique, une chambre de réglage d'air définie à l'intérieur de la base d'ensemble réglage d'air, et une pluralité de premiers évents ;
- une bague de réglage d'air disposée à l'extérieur de la portion tubulaire supérieure de la base d'ensemble réglage d'air, dans laquelle la bague de réglage d'air comprend une pluralité de seconds évents ; et
- une bague d'étanchéité configurée pour empêcher une fuite d'air depuis l'ensemble réglage d'air (8),
- dans laquelle, lorsqu'un utilisateur fait tourner la bague de réglage d'air autour de la portion tubulaire supérieure de la base d'ensemble réglage d'air, et lorsque les emplacements de la pluralité de seconds évents de la bague de réglage d'air concordent avec les emplacements de la pluralité de premiers évents, un écoulement d'air de l'extérieur à la chambre de réglage d'air atteint une capacité maximale, et lorsque l'utilisateur tourne plus avant la bague de réglage d'air autour de la portion tubulaire supérieure, l'écoulement d'air diminue, et lorsque les emplacements de la pluralité de seconds évents de la bague de réglage d'air se désalignent complètement avec les emplacements de la pluralité de premiers évents, l'écoulement d'air s'arrête.

2. Cigarette électronique (10) selon la revendication 1, comprenant en outre un ensemble connecteur électrique (6), dans laquelle l'ensemble connecteur électrique (6) comprend :
une base de connecteur électrique (61) fixée à l'ensemble réglage d'air (8) et adaptée pour connecter l'alimentation électrique aux un ou plusieurs éléments chauffants du vaporisateur céramique, dans laquelle la base de connecteur électrique (61) comprend un filet externe configuré pour connecter électriquement une première borne de l'alimentation électrique de la cigarette électronique à la première borne d'entrée électrique des un ou plusieurs éléments chauffants ;
une électrode (62) configurée pour connecter électriquement la seconde borne de l'alimentation électrique à la seconde borne d'entrée électrique d'un ou de plusieurs éléments chauffants ; et
un bouchon d'isolation (63) positionné entre la base de connecteur électrique (61) et l'électrode (62) pour assurer une isolation entre la première borne et la seconde borne de l'alimentation électrique de la cigarette électronique.

3. Cigarette électronique (10) selon la revendication 1 ou 2, comprenant en outre un interrupteur d'alimentation électrique configuré pour permettre à l'utilisateur d'allumer et d'éteindre l'alimentation électrique de la cigarette électronique (10).

4. Cigarette électronique (10) selon l'une des revendications 1 à 3, comprenant en outre un dispositif de réglage de puissance électrique configuré pour permettre à l'utilisateur de régler la puissance électrique afin de contrôler la vaporisation du liquide à vapoter.

5. Cigarette électronique (10) selon l'une des revendications 1 à 4, comprenant en outre un ensemble embout buccal (9) comportant :
un embout buccal ayant un passage d'air central creux raccordé au passage de vapeur central du vaporisateur céramique (5) et configuré pour fournir du liquide à vapoter vaporisé à un utilisateur ;
un connecteur d'embout buccal ayant une portion inférieure filetée connectée par filets à un support de corps de vaporisateur céramique ; et
une bague d'étanchéité d'embout buccal configurée pour empêcher le liquide à vapoter vaporisé de fuir du connecteur d'embout buccal.

6. Cigarette électronique (10) selon l'une des revendications 1 à 5, dans laquelle le chauffage de vaporisateur (51) comprend un ou plusieurs éléments chauffants configurés pour chauffer le liquide à vapoter en contact direct avec les surfaces du corps de vaporisateur cylindrique (52).

7. Cigarette électronique (10) selon la revendication 6, dans laquelle les un ou plusieurs éléments chauffants comprennent :
un ou plusieurs éléments chauffants à mailles ; et
un ou plusieurs éléments chauffants en spirale.

8. Cigarette électronique (10) selon l'une des revendications 6 ou 7, dans laquelle chacun des un ou plusieurs éléments chauffants comprend :
une première borne d'entrée électrique configurée pour être connectée à une première borne d'une alimentation électrique d'une cigarette électronique ; et
une seconde borne d'entrée électrique configurée pour se connecter à une seconde borne de l'alimentation électrique de la cigarette électronique.

9. Cigarette électronique (10) selon l'une des revendications 1 à 8, dans laquelle le dispositif de stockage de liquide à vapoter comprend un réservoir de stockage de liquide à vapoter à l'extérieur de la surface extérieure.

10. Cigarette électronique (10) selon la revendication 1, dans laquelle le dispositif de stockage de liquide à vapoter comprend un support cylindrique de stockage de liquide à vapoter (53) positionné à l'extérieur de la surface extérieure.
